# EUROPEAN PATENT SPECIFICATION

(11) **EP 2 087 847 B1**
(45) Date of publication and mention of the grant of the patent: **18.07.2012**
(21) Application number: 09001698.1
(22) Date of filing: 06.02.2009
(51) Int. Cl.: A61B 17/70

(54) **Rod connector**
Stangenverbindung
Connecteur de tige

(30) Priority: 07.02.2008 JP 2008028130; 09.01.2009 JP 2009003494
(43) Date of publication of application: 12.08.2009
(73) Proprietor: Showa Ika Kohgyo Co., Ltd., Toyohashi Aichi 441-8026 (JP)
(72) Inventor: Suzuki, Nobumasa, Tokyo 156-0057 (JP); Nohara, Yutaka, Tokyo 135-0091 (JP); Oribe, Kazuya, Aichi 441-8026 (JP)
(74) Representative: Grünecker, Kinkeldey, Stockmair & Schwanhäusser

(56) References cited:
- EP-A- 1 857 064
- US-A1- 2005 277 932
- US-A1- 2006 217 710
- US-A1- 2006 229 611
- US-A1- 2007 173 825
- US-A1- 2008 027 436

## Description

### BACKGROUND OF THE INVENTION

### 1. Field of the Invention

The present invention relates to a rod connector which connects opposed ends of a plurality of rods fixed to a plurality of vertebral bodies (vertebrae) through an implant such as a screw, and, more particularly to a rod connector according to the preamble of claim 1. Such a device is known from EP 1 857 064 A1.

### 2. Description of the Related Art

For example, an implant such as a screw is threadedly inserted and embedded into a plurality of vertebral bodies of such as lumbar vertebrae and thoracic vertebrae, and bone-setting rods are fixed and supported by a head of the screw. In this case, for a portion of the vertebral column including the lumbar vertebrae or the thoracic vertebrae where the vertebral column (spine) is curved, a previously curved rod is used. The Related Art described above are disclosed in the Patent brochures, Japanese Patent Publication No. H7-63485(Laid-Open No. H6-038978) and Japanese Patent No. 2941794(Laid-Open No. 2000-037404).

The curving degree of a spine is different among individuals and the length of a rod to be applied is also different. Therefore, when the rod is previously curved as described in the above patent documents, if the rods are prepared previously, there is a problem that a large number of rods having different curves and lengths need to be prepared, and thus some of the prepared rods may be wasteful.

Further, if a curve of the rod is fixed to and supported on a head of a screw, the rod and the screw may come into contact only partially, and also the fixing screw for fixing the rod to the screw and the rod may come into contact only partially. That is, when a bone-setting rod is fixed and supported by a screw which is threadedly inserted and embedded in a vertebral body, the rod may be brought into an unstable state.

### SUMMARY OF THE INVENTION

The present invention has been achieved with such points in mind.

It therefore is an object of the present invention to provide a rod connector which is previously bent according to the curve of the vertebral column. To achieve the object, a device in accordance with claim 1 is provided.

According to a first aspect of the present invention, there is provided a rod connector for connecting, to each other, opposed ends of a plurality of rods held by a rod holding tool which is fixed to vertebrae (vertebral bodies), wherein a cylindrically formed connector body is previously curved or bent according to the curve of the vertebral column constructed by the vertebrae, both ends of the connector body are provided with a rod insertion hole, rod insertion openings into which the rods are inserted are formed in both end surfaces of the connector body, and the connector body includes screw holes through which fixing screws are threadedly inserted for fixing the rods inserted into the rod insertion hole.

According to a second aspect of the present invention as it depends from the first aspect, in the rod connector, the connector body includes a long hole for visually checking positions of the opposed ends of the rods inserted into the rod insertion hole.

According to a third aspect of the present invention as it depends from the first or the second aspect, in the rod connector, lengths of portions of the rods which are inserted into the connector body can be freely adjusted.

According to any aspect among the first aspect to the third aspect of the present invention, because the rod connector for connecting opposed ends of a plurality of rods is previously curved or bent, when straight rods are connected to each other, the rods are connected to each other in the curved or bent state as a whole. Therefore, if rod connectors having different curved or bent portions are prepared previously, various curves of spines can be dealt with, and the number of previously prepared rods can be smaller than that of a case where rods are previously curved.

Further, a length of a portion of the rod inserted into the rod connector can be adjusted, and therefore rods of various lengths can be dealt with.

### BRIEF DESCRIPTION OF THE ACCOMPANYING DRAWINGS

The above and further objects and novel features of the present invention will more fully appear from the following detailed description when the same is read in conjunction with the accompanying drawings, in which:
Fig. 1 is an explanatory perspective view of a rod connector according to a first embodiment of the present invention;
Fig. 2 is an explanatory plan view of the rod connector according to the first embodiment;
Fig. 3 is an explanatory front view of the rod connector according to the first embodiment;
Fig. 4 is an explanatory diagram showing an arrangement state of the rod connector with respect to a vertebral column (spine); and
Figs. 5A to 5C are explanatory diagrams showing a rod connector not in accordance with the present invention.

### DETAILED DESCRIPTION OF THE PREFERRED EMBODIMENTS

There will be detailed below the preferred embodiments of the present invention with reference to the accompanying drawings. Like members are designated by like reference characters.

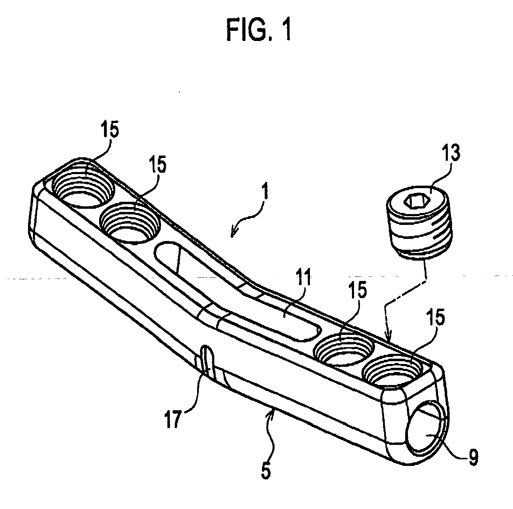

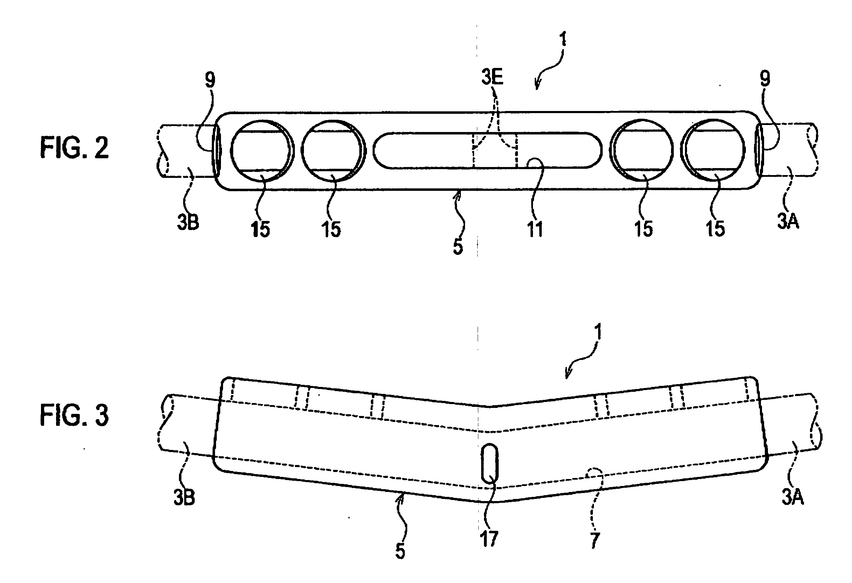

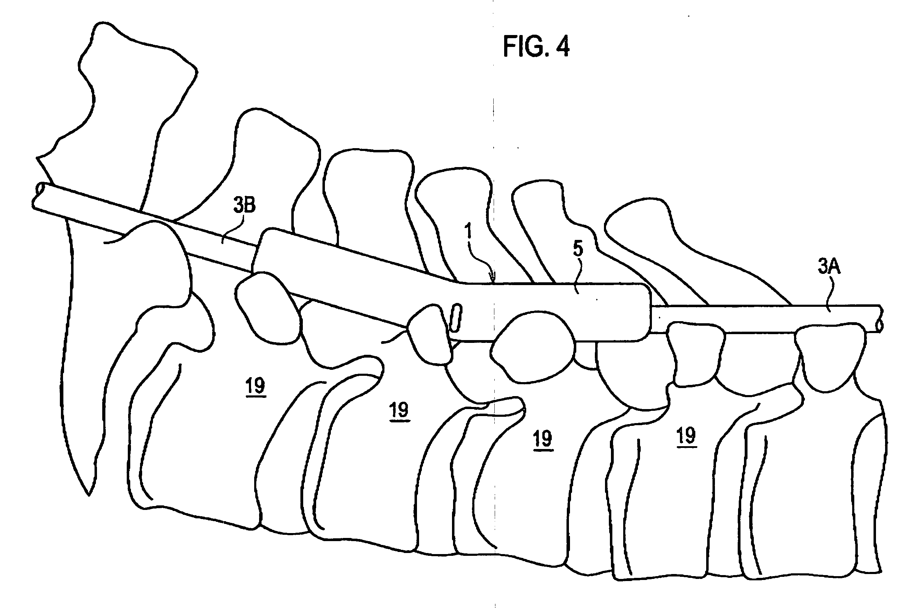

As shown in Fig. 1 and Fig. 2, a rod connector 1 according to a first embodiment of the present invention is used for connecting opposed ends of rods 3A and 3B which are fixed to and held on a vertebral column (a spine) (not shown in Figs. 1 and 2) by a rod holding tool, for example an implant (not shown) such as a screw (see Fig. 4). A connector body S of the rod connector 1 is formed into a cylindrical shape. That is, a rod insertion hole 7 (see Fig. 3) is formed in the connector body 5 such that the rod insertion hole 7 penetrates into the connector body 5, and the rods 3A and 3B are inserted into the rod insertion hole 7. Rod insertion openings 9 are formed in both end surfaces of the connector body 5 through which the rods 3A and 3B are inserted into the rod insertion hole 7.

The connector body 5 is appropriately bent or curved at a central portion thereof in its longitudinal direction into an appropriate direction. A long hole 11 is formed in a central portion of the connector body 5 in its longitudinal direction. Screw holes 15 are formed in both sides of the connector body 5. Fixing screws 13 are threadedly inserted into the screw holes 15 for integrally fixing the rods 3A and 3B which are inserted into the rod insertion hole 7. An engaging recess 17 is formed in the central portion of the connector body 5 in its longitudinal direction, and a holding tool (not shown) for holding the connector body 5 is engaged with the engaging recess 17.

Therefore, when the connector body 5 is held by the holding tool, the balance of the connector body 5 in the longitudinal direction is excellent and the connector body 5 can be handled easily.

In the structure described above, to integrally connect the opposed ends of the rods 3A and 3B which are integrally fixed to the vertebral bodies (vertebrae) 19 (see Fig. 4) by an implant (not shown) such as a screw, the ends of the rods 3A and 3B are inserted into the rod insertion hole 7 from both ends of the connector body 5, and the fixing screws i3 are threadedly inserted into the screw holes 15 to fasten the rods 3A and 3B.

To integrally connect the rods 3A and 3B as described above, the connector body 5 corresponding to the curve is previously placed at the curved portion of the spine, the opposed ends of the rods 3A and 3B are inserted into the connector body 5, the rods 3A and 3B are fixed to the rod holding tool such as a screw which is previously embedded in the vertebral bodies of the spine, the rods 3A and 3B are fastened by the fixing screws 13, and the connector body 5 and the rods 3A and 3B can be integrated to each other.

In this case, positions of the opposed ends 3E of the rods 3A and 3B can be visually checked through the long hole 11 formed in the connector body 5. Therefore, when the lengths of the rods 3A and 3B are insufficient, the rods 3A and 3B may be replaced by longer rods. That is, it is easy to determine whether the rods 3A and 3B to be used have appropriate lengths.

As is already understood, because the insertion positions (inserting lengths) of the rods 3A and 3B with respect to the rod insertion hole 7 of the connector body 5 can be freely adjusted, even if the lengths of the rods 3A and 3B are slightly different from each other, such slight difference can be dealt with. In other words, the same rods 3A and 3B and the same connector body 5 can be used according to the growth of a patient, and it is unnecessary to replace the rods 3A and 3B and the like.

It is also possible to employ the following configuration, i.e., the ends of the rods 3A and 3B are previously inserted into the connector body 5 which corresponds to the curved portion of the spine, the connector body 5 and the rods 3A and 3B are previously integrally connected to each other by the fixing screws 13, the connector body 5 is placed at the curved portion corresponding to the spine, the both ends of the rods 3A and 3B are engaged with the rod holding tool which is previously attached to the vertebral bodies, and the rods 3A and 3B are integrally fixed to the rod holding tool.

As is understood from the above descriptions, because the connector body 5 which is integrally connects the rods 3A and 3B is previously bent or curved appropriately, the connector body 5 which corresponds to the curve of the curved portion can be placed at the curved portion of the spine and the rods 3A and 3B can be connected to each other. Thus, when the rod is placed at a portion where a spine is curved in the longitudinal direction and the lateral direction and in order to fix a plurality of vertebral bodies in the spine to each other by the rod, the connector body 5 can be placed in correspondence with the curve in the longitudinal direction and lateral direction, and a plurality of rods which integrally fix the plurality of vertebral bodies through the rod holding tool can be integrally connected to each other through the connector body 5.

That is, for instance, when a plurality of vertebral bodies of a spine which is curved in the longitudinal direction and the lateral direction are integrally fixed to each other by rods, connector bodies 5 are placed in correspondence with the curved portions of the spine and a plurality of rods are connected to each other without curving a rod according to the curves of the spine, and thus it is possible to deal with complicated curves of a spine easily. That is, it is possible to easily deal with curves of a spine using a plurality of straight rods. Thus, the problems of the conventional technique can be solved.

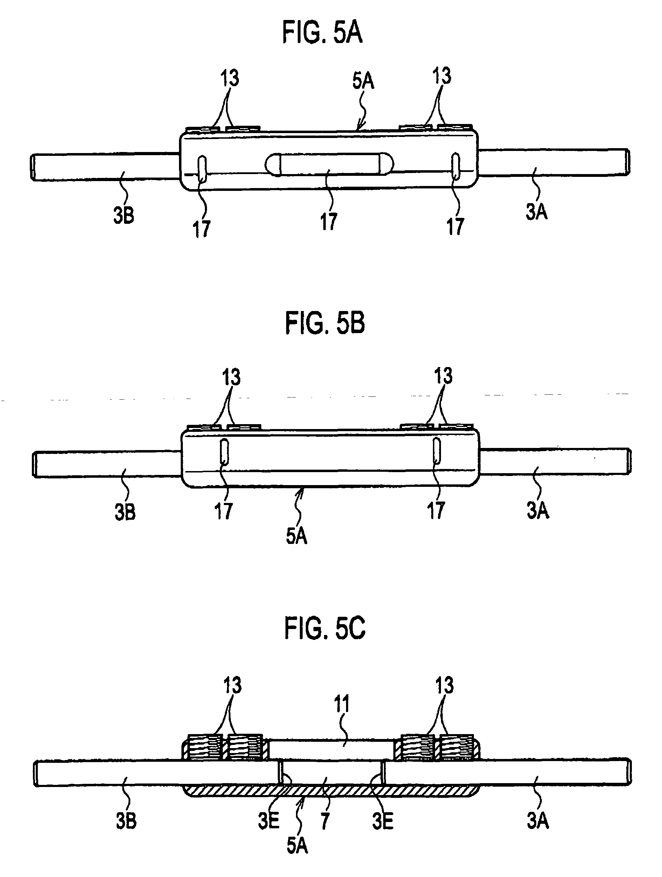

Figs. 5A to 5C show a rod connector not in accordance with the present invention. Constituent elements having the same functions as those of the rod connector in the first embodiment are designated with like reference symbols, and redundant explanations thereof will be omitted. That is, a connector body 5A of the rod connector is formed straightly. Therefore, this rod connector can be used in combination with a previously curved rod, and when rods 3A and 3B are short, this rod connector can be used for straightly connecting the rods 3A and 3B.

Although the invention has been described above by reference to certain embodiments of the invention, the invention is not limited to the embodiments described above. Modifications and variations of the embodiments descried above will occur to those skilled in the art, in light of the above teachings. The scope of the invention is defined with reference to the following claims.

## Claims

1. A rod connector (1) for connecting, to each other, opposed ends (3E) of a plurality of rods (3A, 3B) held by a rod holding tool which is fixed to a vertebra, comprising:
a connector body (5) formed into a cylindrical shape;
a rod insertion hole (7) formed in the connector body (5) in a manner such that the rod insertion hole (7) penetrates into the connector body (5) and such that the rods (3A, 3B) can be inserted into the rod insertion hole (7); and
rod insertion openings (9) formed in both end surfaces of the connector body (5) through which the rods (3A, 3B) can be inserted into the rod insertion hole (7);
wherein the connector body (5) is formed with screw holes (15) through which fixing screws (13) can be threadedly inserted for fixing the rods (3A, 3B) inserted into the rod insertion hole (7);
**characterized in that**
the connector body (5) is previously curved or bent according to the curve of the vertebral column constructed by the vertebrae, such that, when straight rods are connected to each other, the rods are connected to each other in the curved or bent state as a whole.

2. The rod connector (1) according to claim 1,
wherein the connector body (5) includes a long hole (11) for visually checking positions of the opposed ends (3E) of the rods (3A, 3B) inserted into the rod insertion hole (7).

3. The rod connector according to claim 2,
wherein lengths of portions of the rods (3A, 3B) which are inserted into the connector body (5) can be freely adjusted.

## Patentansprüche

1. Stangenverbinder (1) zum Verbinden einander gegenüberliegender Enden (3E) einer Vielzahl von Stangen (3A, 38) miteinander, die von einer an einem Wirbel befestigten Stangenhaltevorrichtung gehalten werden, wobei der Verbinder umfasst:
einen Verbinder-Körper (5), der in einer zylindrischen Form ausgebildet ist;
ein Stangen-Einführloch (7), das in dem Verbinder-Körper (5) so ausgebildet ist, dass sich das Stangen-Einführloch (7) in den Verbinder-Körper (5) hinein erstreckt, und so, dass die Stangen (3A, 3B) in das Stangen-Einführloch (7) eingeführt werden können; und
Stangen-Einführöffnungen (9), die in beiden Endflächen des Verbinder-Körpers (5) ausgebildet sind und über die die Stangen (3A, 3B) in das Stangen-Einführloch (7) eingeführt werden können;
wobei der Verbinder-Körper (5) mit Schraubenlöchern (15) versehen ist, über die Befestigungsschrauben (13) eingeschraubt werden können, um die in das Stangen-Einführloch (7) eingeführten Stangen (3A, 3B) zu befestigen;
**dadurch gekennzeichnet, dass**
der Verbinder-Körper (5) im Voraus entsprechend der Krümmung der durch die Wirbel gebildeten Wirbelsäule so gekrümmt oder gebogen wird, dass, wenn gerade Stangen miteinander verbunden werden, die Stangen insgesamt in dem gekrümmten oder gebogenen Zustand miteinander verbunden sind.

2. Stangenverbinder (1) nach Anspruch 1,
wobei der Verbinder-Körper (5) ein Langloch (11) zum visuellen Prüfen von Positionen der einander gegenüberliegenden Enden (3E) der in das Stangen-Einführloch (7) eingeführten Stangen (3A, 3B) enthält.

3. Stangenverbinder (1) nach Anspruch 2,
wobei Längen von Abschnitten der in den Verbinder-Körper (5) eingeführten Stangen (3A, 3B) frei verstellt werden können.

## Revendications

1. Connecteur de tige (1) pour relier entre elles les extrémités opposées (3E) d'une pluralité de tiges (3A, 3B) maintenues par un outil de maintien de tige qui est fixé à une vertèbre, comprenant :
un corps de connecteur (5) conformé en une forme cylindrique ;
un trou d'insertion de tige (7) formé dans le corps de connecteur (5) de telle manière que le trou d'insertion de tige (7) pénètre dans le corps de connecteur (5) et de telle manière que les tiges (3A, 3B) puissent être insérées dans le trou d'insertion de tige (7) ; et
des ouvertures d'insertion de tige (9) formées dans les deux surfaces d'extrémités du corps de connecteur (5) à travers lesquelles les tiges (3A, 3B) peuvent être insérées dans le trou d'insertion de tige (7) ;
dans lequel le corps de connecteur (5) est muni de trous de vis (15) à travers lesquels des vis de fixation (13) peuvent être insérées par visage pour fixer les tiges (3A, 3B) insérées dans le trou d'insertion de tige (7) ;
**caractérisé en ce que**
le corps de connecteur (5) est préalablement incurvé ou plié conformément à la courbe de la colonne vertébrale construite par les vertèbres, de telle sorte que lorsque les tiges rectilignes sont raccordées entre elles, les tiges sont raccordées entre elles dans l'état incurvé ou plié dans leur totalité.

2. Connecteur de tige selon la revendication 1,
dans lequel le corps de connecteur (5) comporte un long trou (11) pour contrôler visuellement les positions des extrémités opposées (3E) des tiges (3A, 3B) insérées dans le trou d'insertion de tige (7).

3. Connecteur de tige selon la revendication 2,
dans lequel les longueurs des parties des tiges (3A, 3B) qui sont insérées dans le corps de connecteur (5) peuvent être réglées librement.
